# EUROPEAN PATENT APPLICATION

(11) **EP 1 403 256 A1**
(43) Date of publication of application: **31.03.2004**
(21) Application number: 02730769.3
(22) Date of filing: 29.05.2002
(51) Int. Cl.: C07D 235/02

(54) **FUSED IMIDAZOLE DERIVATIVE**

(30) Priority: 05.06.2001 JP 2001169638
(71) Applicant: FUJISAWA PHARMACEUTICAL CO., LTD., Osaka-shi Osaka 541-8514 (JP)
(72) Inventor: SATO, Yoshinari, FUJISAWA PHARMACEUTICAL CO., LTD., Osaka-shi, Osaka 541-8514 (JP); ITO, Harunobu, FUJISAWA PHARMACEUTICAL CO., LTD., Osaka-shi, Osaka 541-8514 (JP); HATORI, Chie, FUJISAWA PHARMACEUTICAL CO., LTD., Osaka-shi, Osaka 541-8514 (JP)
(74) Representative: Gille Hrabal Struck Neidlein Prop Roos
(86) International application number: PCT/JP2002/005246
(87) International publication number: WO 2002/098863

(57) **Abstract**

According to the present invention, fused imidazole derivatives of the general formula: 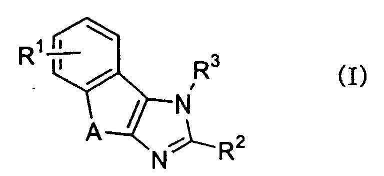 wherein
R¹ is a hydrogen atom, a halogen atom, hydroxy group, a lower alkyl group or a lower alkoxy group,
R² is an aryl group, benzodioxanyl group, or 5-6 membered, monocyclic, unsaturated, heterocyclic group containing nitrogen atom(s) which may be substituted with lower alkyl, trityl or oxo,
R³ is a hydrogen atom or hydroxy group, A is a group represented by the formula : -(CH₂)ₘ - or -O-(CH₂)ₘ- [wherein m is an integer of 1-3]
and their salts are provided.

## Description

### Technical field

This invention relates to fused imidazole derivatives, and in more detail to fused imidazole derivatives having neuropeptide Y-antagonistic activity.

### Background of technique

Neuropeptide Y (hereafter referred to as NPY) is widely distributed in the central and peripheral nerve systems, and is well known to be a neurotransmitter which plays an important role in the management of a various diseases.

Recently, it has become evident that many kinds of NPY receptors exist and exhibit a different physiological and pharmacological activities. For example, Y5 receptors are present as one of the cloned substances, which are characterized as a sub-type of new NPY receptors.

As diseases associated with Y5 receptors, for example, obesity, ingestion disorder and others are generally known. Compounds which function as antagonists of sub-type of the receptors are disclosed in Official Gazette W097/ 19862 and Official Gazette W099/01128.

Therefore, the development of compounds functioning as antagonists of sub-type of Y5 receptors is considered to be useful in the treatment of the diseases such as obesity, ingestion disorder and others, and even in the prevention and treatment of diabetes, hyperlipemia, hypertension, arteriosclerosis and others originating from those diseases.

### Disclosure of invention

According to the present invention, fused imidazole derivatives of the general formula: 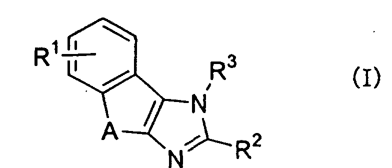 wherein
R¹ is a hydrogen atom, a halogen atom, hydroxy group, a lower alkyl group or a lower alkoxy group,
R² is an aryl group, benzodioxanyl group, or 5-6 membered, monocyclic, unsaturated, heterocyclic group containing nitrogen atom(s) which may be substituted with lower alkyl, trityl or oxo,
R³ is a hydrogen atom or hydroxy group, and
A is a group represented by the formula : -(CH₂)ₘ - or -O-(CH₂)ₘ- [wherein m is an integer of 1-3]
and their salts are provided.

### Best mode for carrying out the invention

Various definitions in the fused imidazole derivatives represented by the general formula (I) of the present invention are explained in detail in the following.

As the halogen atom, fluorine, chlorine, bromine and iodine are mentioned, among which fluorine or chlorine is preferable.

As a lower alkyl moiety in the lower alkyl group and the lower alkoxy group, a linear or branched one having 1-6 carbon atoms such as methyl, ethyl, propyl, isopropyl, n-butyl, isobutyl, sec-butyl, tert-butyl, pentyl, isopentyl, hexyl may be mentioned. Preferable lower alkyl moiety is the one having 1-4 carbon atoms such as ethyl, ethyl, propyl, isopropyl, n-butyl, isobutyl, sec-butyl or tert-butyl, and more preferable one is methyl or ethyl.

As a lower alkoxy group, a linear or branced one having 1-6 carbon atoms such as methoxy, ethoxy, propoxy, isopropoxy, n-butoxy, isobutoxy, sec-butoxy, tert-butoxy, pentyloxy, isopentyloxy, hexyloxy may be mentioned. Preferable lower alkoxy is the one having 1-4 carbon atoms such as methoxy, ethoxy, propoxy, isopropoxy, n-butoxy, isobutoxy, sec-butoxy or tert-butoxy, and more preferable one is methoxy or ethoxy.

The group R¹ may exit on any position of the benzene ring of the formula (I), and may exist, for example, on a position as shown in the following.

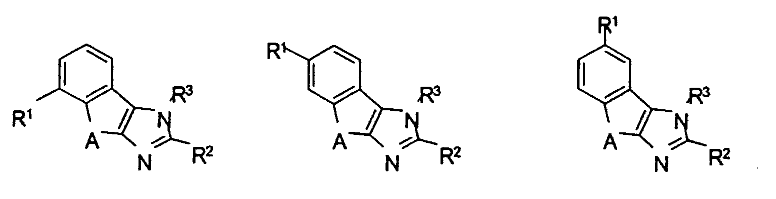

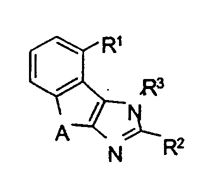

Among the above, it is preferable to be substituted on the position as shown in the following.

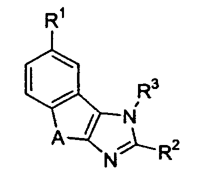

As an aryl group, for example, phenyl, naphthyl and the like may be mentioned, among which phenyl is preferable.

As a 5-6 membered, monocyclic, unsaturated heterocyclic group containing nitrogen atom(s), heterocyclic group containing 1-4 nitrogen atoms, for example, heterocyclic group containing one nitrogen atoms such as pyrrolyl or pyridyl, heterocyclic group containing 2 nitrogen atoms such as pyrazolyl, imidazolyl, pyridazinyl, pyrimidinyl or pyrazinyl, heterocyclic group containing 3 nitrogen atoms such as triazolyl, heterocyclic group containing 4 nitrogen atoms such as tetrazolyl may be mentioned.

Among the above, preferable ones are monocyclic, unsaturated, heterocyclic groups containing 1 or 2 nitrogen atoms, and more preferable ones are imidazolyl, pyridyl and pyridazinyl.

These monocyclic, unsaturated, heterocyclic group containing nitrogen atom(s) may be substituted with lower alkyl group, trityl group and/or oxo group.

Preferably, the fused imidazole derivatives (I) of the present invention are the compounds of the formula (I) wherein R¹ is a hydrogen atom, a halogen atom, hydroxy group, a lower alkyl group or a lower alkoxy group, R² is an aryl group, benzodioxanyl group or a 5-6 membered, monocyclic, unsaturated heterocyclic group containing nitrogen atom(s) which may be optionally substituted with lower alkyl, trityl or oxo, R³ is a hydroen atom or hydroxy group, and A is a group represented by the formula: -(CH₂)₂-, -(CH₂)₃- or-O-(CH₂)₂-.

More preferably, the fused imidazole derivatives (I) of the present invention are the compounds of the formula (I) wherein R¹ is a hydrogen atom, R² is an aryl group and then R³ is a hydrogen atom or hydroxy group, when A is a group represented by the formula:

- O-(CH₂)₂-

The salts of the fused imidazole derivatives (I) may be pharmaceutically acceptable salts, for example, a salt with an inorganic acid such as hydrochloric acid, hydrofluoric acid, hydrobromic acid, hydroiodic acid, sulfuric acid, nitric acid, phosphoric acid, boric acid, a salt with an organic acid such as acetic acid, trifluoroacetic acid, formic acid, tartaric acid, citric acid, fumaric acid, toluenesulfonic acid, methanesulfonic acid, and the like.

Tautomers of the fused imidazole derivatives (I) are included in the scope of the present invention.

Although an isomer of the tautomers due to transfer of a double bond in a molecule is shown as the formula (I), the other tautomers are included in the fused imidazole derivatives represented by the formula (I).

The fused imidazole derivatives and their salts of the present invention can be prepared by the following methods.

### Preparation Method 1:

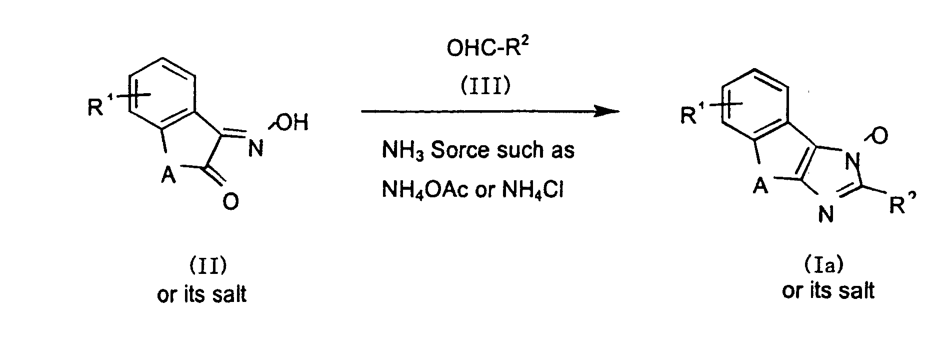

### Preparation Method 2

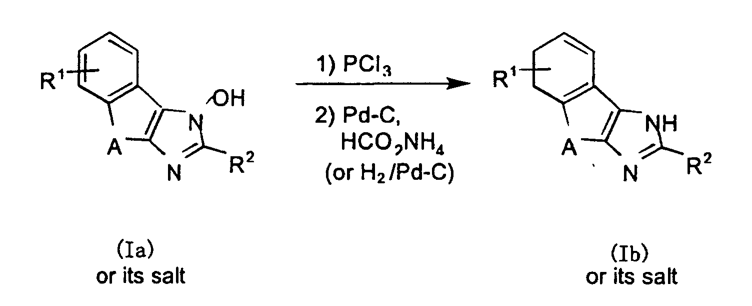

wherein
R¹, R² and A are as defined above.

A compound (Ia) or its salt can be obtained by reacting a compound (II) or its salt with an aldehyde (III).

The reaction is usually carried out at room temperature - under heating at about 120 °C in the presence of a salt which can become an ammonia source in a solvent such as ammonium acetate, ammonium chloride, or the like. Water, dioxane, alcohol such as methanol, ethanol, or a mixture thereof can be used as a solvent.

A compound (Ib) or its salt can be obtained by reacting a compound (Ia) or its salt with a reducing agent such as phosphorus (III) chloride, titanium trichloride, etc., by catalytic reduction using Pd-C catalyst, or by treating a compound (Ia) or its salt with a catalytic amount of Pd-C and ammonium formate.

The reaction of a compound (Ia) or its salt with phosphorous (III) chloride is carried out by reacting the compound (Ia) or its salt with 1-2.5 equivalent of phosphorus (III) chloride in a solvent at room temperature for about 2-15 hours. A solvent which does not adversely affect the reaction such as dimethylformamide can be as a solvent.

The catalytic reduction using Pd-C catalyst is usually carried out under normal pressure or increased pressure (3 atmospheric pressure) for 1-3 days. The reduction with Pd-C and ammonium formate is usually carried out in a solvent at room temperature - under heating with reflux for 1 to 5 days. A solvent which does not adversely affect the reaction such as tetrahydrofuran, dioxane toluene, methanol, ethanol, etc. is used as a solvent.

An unsaturated compound (group A contains a group-CH = CH-) which may be occasionally produced as a side product in the reduction reaction using phosphorus (III) chloride can be converted to an object compound (Ib) or its salt by catalytic reduction or by treating with Pd-C and ammonium formate.

A compound (II) or its salt to be used as a starting material in Preparation Method 1 can be prepared according to the following process. 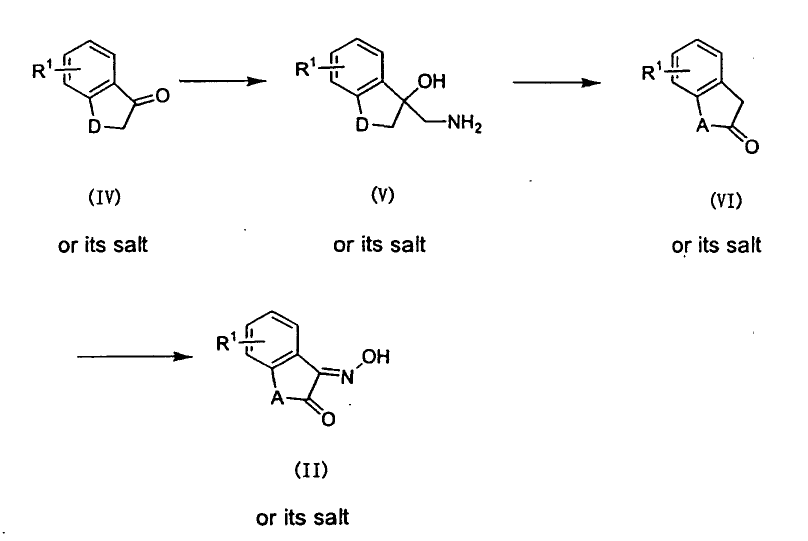 wherein
R¹ and A are as defined above, and D is a group represented by the formula : - (CH₂)ₘ₋₁ -or -O-(CH₂)ₘ₋₁-, in which m is as defined above.

A compound ( V ) or its salt can be obtained by reacting a compound (IV) or its salt with a catalytic amount of Zinc iodide and trimethylsilylnitrile and then by reacting the resultant cyanohydrin derivative with a reducing agent such as lithium aluminium hydride.

Then, a compound (V) or its salt reacted with sodium nitrite under acidic condition to give a compound (VI) or its salt.

A compound (VI) or its salt is then reacted with isoamyl nitrite to give a compound (II) or its salt.

Compounds (IV) or their salts which are used as stating materials in the above methods are known compounds and can be prepared by known methods.

Compounds (II) or their salts can be prepared, for example, by the methods described in the following Preparations 1-10 or similar methods thereto.

The synthetic intermediates and object compounds in the above methods can be isolated the reaction mixture and purified by known methods, for example, extraction, condensation, neutralization, filtration, recrystallization, various chromatography, and the like.

The intermediates may be used in the subsequent process without isolation.

Fused imidazole derivatives (I) and their salts have antagonism against NPY receptors, especially NPY-Y5 receptors. Accordingly, these derivatives and their salts are useful for treatment of the following diseases: ingestion disorders such as obesity, anorexia nervosa, cibophobia, and nervous polyphagia; abnormal symptoms such as sexual/reproduction disorder, hypogonadism, inflammation, asthma, depression, epileptic seizure, vascular spasm, hypertension, cerebral apoplexy, congestive cardiac insufficiency, shock, cardiomegalia, angina, cardiac infarction, arrythmia, hyperlipemia, peripheral vascular disorder, arteriosclerosis, renal insufficiency, cerebral infarction, nerve degeneration, apoplexy, anxiety, allophasis, dimentia, pain, gastrointestinal dysfunction, urinary incontinence, Crohn's disease, and sleep disturbance; hormone-releasing disorders such as lutenizing hormone, growth hormone, insulin, and luteotropic hormone.

According to the present invention, a phramceutical composition comprising a fused imidazole derivative ( I ) or its salt as an effective ingredient is provided. The pharmaceutical composition of the present invention comprised pharmaceutically acceptable carries such as organic or inorganic, solid, semisolid or liquid excipients suitable for oral, parenteral or external administration, and can be formulated by methods known in the present technical field into a pharmaceutical preparation such as powder, capsules, tablets, fine granules, granules, suppositories, solution, lotion, inhalation, eyedrop, nosedrop, suspension, emulsion, ointment, gel, or the like. Conventional additive(s) such as auxiliary agent, stabilizing agent, wetting agent, emulsifying agent, buffering agent, etc. may be optionally added to the above preparation.

Medical components of the present invention can be used for the above-mentioned diseases, and are especially suitable for prevention and treatment of obesity, diabetes, hyperlipemia, hypertension and arteriosclerosis. The dosage of a condensated imidazole derivative (I) or its salt as active ingredient varies according to the kinds of the active ingredients, and the patient's age and symptom. A dose of about 0.1 to 1000 mg of the compound is served to an adult, and so the compound is generally given to an adults in a daily dose of 0.1 to 1000 mg.

### Example

Following Preparations and Examples are given for illustrating the present invention in more detail and should not be understood to limit the scope of this invention.

Each abbreviation used in the following Examples has the following meaning, respectively.
- HOBt :: 1-hydroxybenzotriazole
- WSC: : N-dimethylaminopropyl-N'-ethylcarbodiimide
- DMF :: N,N-dimethylformamide
- THF: : tetrahydrofuran
- DMSO :: dimethylsulfoxide
- TFA: : tetrafluoroacetic acid

### Preparation 1

Trimethylsilylnitrile (95.6ml) was added dropwise to a mixture of 7-methoxytetralone (100g) and zinc iodide (3.62g) in dichloromethane (560ml), and the mixture was stirred overnight at room temperature. An aqueous solution (250ml) saturated with sodium bicarbonate was added to the reaction mixture with stirring. The aqueous layer was separated and extracted with dichloromethane. The extract was combined with the separated organic layer and washed with water twice. The solution was dried over magnesium sulfate and the solvent was removed under vacuum to give an oily intermediate, 1-cyano-1-trimethylsilyloxy-7-methoxytetralin (175.0g). A solution of the oily substance obtained in the above in THF (500ml) was added dropwise to a suspension of lithium aluminium hydride (43.07g) in THF (500ml) with stirring at 15-25°C in 3 hours. The mixture was stirred for 4 hours under the same conditions. Sodium fluoride (199.2g) was added to the reaction mixture. To the mixture was added carefully a mixed solvent of water (85.4ml) and THF (500ml) with stirring under cooling in an ice-water bath. The resultant precipitates were removed by filtration and washed. The filtrate and washing were combined and distilled to dryness under reduced pressure to give oily 1-aminomethyl-1-hydroxy-7-methoxytetralin (115.27g). This product was used in the following reaction without purification.
Mass(APCI): m/z=208(M⁺+1), 190(M⁺+1-H₂O) ;
¹H-NMR(CDCl₃) δ : 1.4-1.7(2H, br.), 1.7-2.1(4H, m), 2.7- 2.8(2H, m), 2.88(2H, ABq, J=13.2Hz, 19.4Hz), 3.79(3H, s ), 6.75(1H, dd, J=2.7Hz, 7.4Hz), 7.00(1H, d, J=7.4Hz), 7.07(1H, d, J=2.7Hz).

### Preparation 2

The following compounds (1)-(4) were prepared according to the similar method to the above Preparation 1.
(1) 1-Aminomethyl-1-hydroxytetralin
   Physicochemical Property of this product was not measured because it was used in the subsequent reaction without purification.
(2) 4-Aminomethyl-4-hydroxy-2,3-dihydro-1(4H)-benzopyran
   Mass(APCI): m/z=162(M⁺+1-H₂O);
   ¹H-NMR(CDCl₃) δ : 2.04(2H, br.s), 2.07-2.29(2H, m), 3.05(2H, ABq, J=13.1Hz, 26.6Hz), 4.17-4.40(2H, m), 6.87(1H, d, J=7.4Hz), 6.97(1H, t, J=7.4Hz), 7.23(1H, t, J=7.4Hz), 7.46(1H, d, J=7.4Hz).
(3) 1-Aminomethyl-1-hydroxy-7-flluorotetralin
   Mass(APCI): m/z=177(M⁺-H₂O);
   ¹H-NMR(CDCl₃) δ : 1.42(2H, br.s), 1.72-2.08(4H, m), 2.75(2H, m), 2.86(2H, ABq, J=13.1Hz, 26.6Hz), 3.52(1H, br.s), 6.86(1H, td, J=2.7Hz, 8.4Hz), 7.03(1H, dd, J=5.8Hz, 8.4Hz), 7.23(1H, dd, J=2.7Hz, 10.2Hz).
(4) 1-Aminomethyl-1-hydroxy-7-chlorotetralin
   Mass(APCI): m/z=194(M⁺+1-H₂O);
   ¹H-NMR(CDCl₃) δ : 1.25(2H, br.s), 1.6-2.1(4H, m), 2.7-3.0(4H, m), 3.58(1H, br.s), 7.0(1H, d, J=8.2Hz), 7.13(1H, dd, J=2.2Hz, 8.2Hz), 7.52(1H, d, J=2.2Hz).

### Preparation 3

An aqueous solution (145ml) of sodium nitrite (29.78g) was added dropwise to a solution of 1-aminomethyl-1-hydroxy-7-methoxytetralin (71.40g) in 10% acetic acid (1.4L) with stirring at 0-5°C in 2.5 hours. The mixture was stirred for 3 hours under the same condition. Ethyl acetate (1.5L) was added to the reaction mixture with stirring. Separated aqueous layer was extracted with ethyl acetate and the extract was combined with the separated organic layer. The mixture was washed with water, aqueous solution of sodium bicarbonate and water successively. After drying over magnesium sulfate, solvent was removed under vacuum to give an oily product (51.60g). The oily product was allowed to stand and the resultant precipitates were ground and collected by filtration to give the first crystals of 3-methoxy-6,7,8,9-tetrahydro-5H-benzo[a] cycloheptan-6-one (28.56g).

The filtrate obtained in the above was distilled, and the resultant oily product was subjected to column chromatography on silica gel eluting with a mixture of n-hexane and ethyl acetate (10:1). The fractions containing the desired product were combined and distilled under vacuum to give the second crystals of the above compound (13.90g).
Mass(APCI):m/z=191(M⁺+1) ;
¹H-NMR(CDCl₃) δ :1.89-2.03(2H, m), 2.56(2H, t, J=7.0Hz), 2.85-2.92 (2H, m), 3.69(2H,s), 3.79(3H, s), 6.7-6.77(2H, m), 7.06(1H, d, J=8.9Hz).

### Preparation 4

Following compounds (1)-(4) were prepared by similar method to Preparation 3.
(1) 6,7,8,9-tetrahydro-5H-benzo [a]cycrohepten-6-one
   Mass(APCI): m/z=161(M⁺+1) ;
   ¹H-NMR(CDCl₃) δ : 1.9-2.1(2H, m), 2.57(2H, t, J=7.0Hz), 2.94(2H, t, J = 7.0Hz), 3.73(2H, s), 7.1-7.3(4H, m).
(2) 4-Oxo-2,3,4,5-tetrahydro-1-benzoxepine
   Mass(API-ES/Negative): m/z=161(M⁺-1);
   ¹H-NMR(CDCl₃) δ : 2.86(2H, t, J=5.9Hz), 3.85(2H, s), 4.38(2H, t, J=5.9Hz), 4.17-4.40(2H, m), 6.99-7.23(4H, m).
(3) 3-Fluoro-6,7,8,9-tetrahydro-5H-benzo [a] cycrohepten-6-one
   Mass(APCI): m/z=179(M⁺+1);
   ¹H-NMR(CDCl₃) δ : 1.98(2H, quintet, J=6.6Hz), 2.57(2H, t, J=6.6Hz), 2.92(2H, t, J=6.6Hz), 3.69(2H, s), 6.85-6.94(2H, m), 7.07-7.15(1H, m).
(4) 3-Chloro-6,7,8,9-tetrahydro-5H-benzo [a] cyclohepten-6-one
   Mass(APCI): m/z=195(M⁺+1);
   ¹H-NMR(CDCl₃) δ : 1.99(2H, quintet, J=6.9Hz), 2.57(2H, t, J=6.9Hz), 2.91(2H, t, J=6.9Hz), 3.68(2H, s), 7.06-7.21(3H, m).

### Preparation 5

A solution of isoamyl (6.45ml) in THF (10ml) was added dropwise to a solution of 3-methoxy-6,7,8,9-tetrahydro-5H-benzo [a] cyclohepten-6-one (7.61g) in a mixed solvent of THF (25ml), water (20ml) and conc. hydrochloric acid (13.3ml) with stirring under cooling at 5-10°C. The mixture was stirred under the same condition for 2 hours and then at room temperature for 2 hours. After removing THF, the remaining mixture was extracted with ethyl acetate, and the extract was washed with water, aqueous solution of sodium bicarbonate and water in turn and then dried over magnesium sulfate. The solvent was removed under vacuum to give orange colored oily product (14.09g). The product was subjected to column chromatography on silica gel eluting with a mixed solvent of chloroform and methanol (50:1). Fractions containing the desired product were combined and distilled under vacuum to give orange oily 3-methoxy-6,7,8,9-tetrahydro-5H-benzo [a] cyclohepten-5-hydroxyimino-6-one (8.20g).
Mass(APCI):m/z=220(M⁺+1) ;
¹H-NMR(CDCl₃) δ :2.04(2H, quintet, J=7.1Hz), 2.58(2H, t, J=7.1Hz), 2.76(2H, t, J=7.1Hz), 3.83(3H,s), 6.94(1H, dd, J=2.7Hz, 8.3Hz), 7.17(1H, d, J=8.3Hz), 7.37(1H, d, J=2.7Hz), 10.79(1H, br.s).

### Preparation 6

Following compounds (1) and (2) were prepared by a similar method to Preparation 5.
(1) 6,7,8,9-Tetrahydro-5H-benzo [a] cyclohepten-5-hydroxyimino-6-one
   Mass(APCI): m/z=161(M⁺+1);
   ¹H-NMR(CDCl₃) δ ; 2.07(2H, quintet, J=7.1Hz), 2.58(2H, t, J=7.1Hz), 2.83(2H, t, J =7.1Hz), 7.3-7.5(4H, m), 7.7-7.85(1H, m).
(2) 3-Fluoro-5-hydroxyimino-6,7,8,9-tetrahydro-5H-benzo [a] cyclohepten-6-one
   Mass(APCI): m/z=208(M⁺+1);
   ¹H-NMR(CDCl₃) δ : 1.88(1H, br.s), 2.07(2H, quintet, J=7.1Hz), 2.59(2H, t, J=7.1Hz), 2.80(2H, t, J=7.1Hz), 7.05-7.28(2H, m), 7.51-7.57(1H, m).

### Preparation 7

A solution of isiamyl nitrite (1.83ml) in THF (4ml) was added dropwise to a solution of 7-methoxy-2-tetralone (2.0g) in a mixed solvent of THF (4ml), water (6ml) and 12N-HCl (3.75ml) with stirring at -20°C. The mixture was stirred under the same condition for 2 hours and then neutralized with powdered sodium bicarbonate at -15°C. Water was added to the mixture and the mixture was extracted with ethyl acetate. The extract was washed with water twice and dried over magnesium sulfate. Solvent was removed under vacuum, and the resultant red colored oily product was subjected to column chromatography on silica gel eluting with chloroform. Fractions containing the desired product were combined and distilled under vacuum to give amorphous 7-methoxy-1-hydroxyimino-2-tetralone (0.50g).
¹H-NMR(CDCl₃) δ : 2.81(2H, t, J=6.5Hz), 3.06(2H, t, J=6.5Hz), 3.85(3H, s), 6.97(1H, dd, J=2.7Hz, 8.4Hz), 7.22(1H, d, J=8.4Hz), 8.31(1H, d, J=2.7Hz).

### Preparation 8

3-(4-Fluorobenzoyl)propionic acid (9.81g) was dissolved in acetic acid (50ml). 70% Perchloric acid (0.9ml) was added to the solution, and the mixture was hydrogenated with hydrogen (2 atmospheric pressure) in the presence of 10% palladium on carbon. The catalyst was removed by filtration with Celite, and the filtrate was distilled under vacuum. The resultant residue was dissolved in ethyl acetate, washed with water repeatedly and dried over magnesium sulfate. The solvent was removed under vacuum to give oily 4-(4-fluorophenyl)butyric acid (9.09g). This product was used in the subsequent reaction without further purification.
Mass(APCI): m/z=182(M⁺), 181(M⁺-1);
¹H-NMR(CDCl₃) δ : 1.94(2H, quintet, J=7.3Hz), 2.37(2H, t, J=7.3Hz), 2.65(2H, t, J=7.3Hz), 6.86-7.25(4H, m).

### Preparation 9

Polyphosphoric acid (newly prepared from phosphorous pentoxide (45g) and phosphoric acid (45ml)) was added to 4-(4-fluorophenyl) butyric acid (9.09g) with stirring at 10°C. The mixture was stirred under the same condition for 1.5 hours. Ice-water was poured into the reaction mixture and extracted with ethyl acetate twice. The extracts were combined, washed with an aqueous solution of sodium bicarbonate, water and aqueous solution of sodium chloride and then dried over magnesium sulfate. The solvent was removed, and the resultant oily product (7.96g) was subjected to column chromatography on silica gel eluting with chloroform. Fractions containing the desired compound were combined and distilled under vacuum to give 7-fluoro-2-tetralone (4.75g) as colorless solid.
Mass(APCI): m/z=165(M⁺+1);
¹H-NMR(CDCl₃) δ : 2.14(2H, quintet, J=6.4Hz), 2.66(2H, t, J=6.4Hz), 2.94(2H, t, J=6.4Hz), 7.12-7.28(3H, m), 7.69(1H, dd, J=2.7Hz, 9.2Hz).

### Preparation 10

A solution of 7-fluoro-1-tetralone (4.75g) and zinc iodide (160mg) in dichloromethane (55ml) was cooled in an ice bath. To the solution was added dropwise trimethylsilylnitrile (4.87ml) with stirring, and the mixture was stirred under the same condition for an hour and then at room temperature overnight. To the mixture was added an aqueous solution of sodium bicarbonate, and then dichloromethane was removed under vacuum. The resultant aqueous residue was extracted with ethyl acetate twice, and the extract was washed with water and an aqueous solution of sodium chloride, and then dried over magnesium sulfate. The silvent was removed to give 1-cyano-1-trimethylsilyloxy-7-fluorotetralin (7.25g) as an red colored oily product. This product was used in the successive reaction without further purification.
¹H-NMR(CDCl₃) δ : 0.28(9H, s), 1.92-2.43(4H, m), 2.79(2H, t, J=6.2Hz), 6.93-7.12(3H, m), 7.33(1H, dd, J=2.6Hz, 9.5Hz).

### Example 1

A solution of 3-methyoxy-6,7,8,9-tetrahydro-5H-benzo [a] cyclohepten-5-hydroxyimino-6-one (3.20g), pyridine-4-aldehyde (1.72g) and ammonium acetate (3.40g) in a mixed solvent of 1, 4-dioxane (60ml), ethanol (20ml) and water (20ml) was stirred at 100°C for 2 hours. The reaction mixture was cooled to room temperature, and then organic solvent was removed under vacuum. To the residue was added an aqueous solution of sodium bicarbonate, and the mixture was extracted with ethyl acetate three times. The extract was washed with water and an aqueous solution of sodium chloride, and then dried over magnesium sulfate. Solvent was removed under vacuum, and the resultant oily product was subjected to column chromatography on silica gel eluting with a mixed solvent of chloroform and methanol (40:1). Fractions containing the desired product were combined and distilled under vacuum to give amorphous 9-methoxy-2-(pyridin-4-yl)-5,6-dihydro-4H-1,3-diazabenzo [e] azulen-1-ol (2.89g).
Mass(APCI): m/z=308(M⁺+1) ;
¹H-NMR(CDCl₃) δ :1.92 (2H, br.quintet, J = 7.0Hz), 2.47(2H, br.t, J = 7.0Hz), 2.68 (2H, t, J = 7.0Hz), 3.2 - 4.0(1H, br.), 3.74(3H, s), 6.70(1H, dd, J = 2.6Hz, 8.3Hz), 7.03(1H, d, J = 8.3Hz), 7.70(1H, d, J = 2.6Hz), 7.88( 2H,J = 6.2Hz), 8.11(2H, d, J = 6.2Hz).

### Example 2

Following compounds (1)-(13) were prepared according to similar method to Example 1.
(1) 9-Methoxy-2-phenyl-5,6-dihydro-4H-1,3-diazabenzo [e] azulen-1-ol
   Mass(APCI): m/z=307(M⁺+1) ;
   ¹H-NMR(CDCl₃) δ ; 1.65-1.80 (2H, m), 2.25-2.40(2H, m), 2.44(2H, t, J = 7.05Hz), 2.68 (2H, t, J = 7.0Hz), 3.62(3H, s), 4.80(1H, br), 6.59(1H, dd, J = 2.6Hz, 8.3Hz), 6.89(1H, d, J = 8.3Hz), 6.95-7.08(3H, m), 7.79(2H,J = 7.8Hz), 7.89(1H, d, J = 2.6Hz).
(2) 9-Methoxy-2-(pyridin-3-yl)-5,6-dihydro-4H-1,3-diazabenzo [e] azulen-1-ol
   Mass(APCI): m/z=308(M⁺+1);
   ¹H-NMR(DMSO-d₆) δ : 1.9-2.0(2H, m), 2.6-2.7(2H, m), 2.86(2H, t, J = 7.3Hz), 3.77(3H, s), 6.79(1H, dd, J = 2.7Hz, 8.3Hz), 7.18(1H, d, J = 8.3Hz), 7.50(1H, dd, J=4.7Hz, 8.3Hz), 7.58(1H, br.s), 8.40(1H, d, J=7.9Hz), 8.58(1H, dd, J =1.6Hz, 4.8Hz), 9.23(1H, d, J = 1.6Hz), 12.0(1H, br).
(3) 9-Methoxy-2-(pyridin-2-yl)-5,6-dihydro-4H-1,3-diazabenzo [e] azulen-1-ol
   Mass(APCI): m/z=308(M⁺+1);
   ¹H-NMR(DMSO-d₆) δ : 1.8-2.0(2H, m), 2.6-2.7(2H, m), 2.8-3.0(2H, m), 3.77(3H, s), 6.79(1H, dd, J = 2.7Hz, 8.3Hz), 7.16(1H, d, J = 7.9Hz), 7.3-7.6(2H, m), 8.01(1H, br.s), 8.67(1H, d, J=4.5Hz), 9.23(1H, br.s), 12.99(1H, br).
(4) 8-Methoxy-2-phenyl-4,5-dihydronaphtho [1,2-d] imidazol-1-ol
   Mass(APCI): m/z=293(M⁺+1);
   ¹H-NMR(CDCl₃) δ : 2.26(2H, t, 7.6Hz), 2.59(2H, t, J=7.6Hz), 3.56(3H, s), 6.57(1H, dd, J = 2.6Hz, 8.3Hz), 6.9-7.05(4H, m), 7.7-7.85(3H, m).
(5) 2-Phenyl-5,6-dihydro-4H-1,3-diazabenzo [e] azulen-1-ol
   Mass(APCI): m/z=277(M⁺+1);
   ¹H-NMR(DMSO-d₆) δ : 1.6-1.85(2H, m), 2.2-2.5(4H, m), 6.95-7.1(6H, m), 7.80(2H, d, J=7.8Hz), 8.13(1H, m), 8.44(1H, br.)
(6) 2-(Pyridin-2-yl)-5,6-dihydro-4H-1,3-diazabenzo [e] azulen-1-ol
   Mass(APCI): m/z=278(M⁺+1);
   ¹H-NMR(DMSO-d₆) δ :1.85-2.1(2H, m), 2.7-3.05(4H, m), 7.08-8.8(9H, m).
(7) 2-(Pyridin-3-yl)-5,6-dihydro-4H-1,3-diazabenzo [e] azulen-1-ol
   Mass(APCI): m/z=278(M⁺+1);
   ¹H-NMR(DMSO-d₆) δ : 1.9-2.1(2H, m), 2.65-2.8(3H, m), 2.8-2.95(2H, m), 7.2-7.35(3H, m), 7.51(1H, dd, J=4.9Hz, 8.0Hz), 7.94(1H, d, J=7.1Hz), 8.37(1H, d, J=8.1Hz), 8.57(1H, dd, J=1.5Hz, 4.8Hz), 9.21(1H, s), 11.88(1H, s).
(8) 2-(Pyridin-4-yl)-5,6-dihydro-4H-1,3-diazabenzo [e] azulen-1-ol
   Mass(APCI): m/z=278(M⁺+1);
   ¹H-NMR(DMSO-d₆) δ : 1.9-2.1(2H, m), 2.65-2.75(3H, m), 2.8-2.95(2H, m), 7.2-7.4(3H, m), 7.85-8.15(3H, m), 8.65(2H, dd, J=1.4Hz, 4.7Hz), 12.03(1H, s).
(9) 2-(1,4-Benzodioxan-6-yl)-5,6-dihydro-4H-1,3-diazabenzo [e] azulen-1-ol
   Mass(APCI): m/z=335(M⁺+1);
   ¹H-NMR(CDCl₃) δ :1.65-1.85(2H, m), 2.3-2.55(4H, m), 3.85-4.1(4H, m), 6.51(1H, d, J=8.5Hz), 6.9-7.1(3H, m), 7.2-7.35(2H, m), 7.45(1H, d, J=2.0Hz), 8.05-8.2(1H, m).
(10) 2-(1-Isopropyl-6-oxo-1, 6-dihydropyridazin-3-yl)-5,6-dihydro-4H-1,3-diazabenzo [e] azulen-1-ol
   Mass(APCI): m/z=337(M⁺+1);
   ¹H-NMR(CDCl₃) δ : 1.44(6H, d, J=6.7Hz), 1.65(1H, br.s), 2.05-2.20(2H, m), 2.7-2.82(2H, m), 2.95(2H, t, J=7.3Hz), 5.39(1H, heptet, J=6.7Hz), 7.04-7.37(4H, m), 7.95-8.15(4H, m).
(11) 2-(Imidazol-3-yl)-5,6-dihydro-4H-1,3-diazabenzo [e] azulen-1-ol
   Mass(APCI): m/z=267(M⁺+1);
   ¹H-NMR(CDCl₃) δ :1.85-2.05(2H, m), 2.6-2.8(4H, m), 2.7-3.5(2H, br.), 7.1-7.25(3H, m), 7.40(1H, s), 7.99(1H, s), 8.50(1H, d, J=7.0Hz).
(12) 2-Phenyl-4,5-dihydro-1H-[1] benzoxepino [4,5-d] imidazol-1-ol
   Mass(APCI): m/z=279(M⁺+1);
   ¹H-NMR(CDCl₃) δ :2.70(2H, t, J=5.2Hz), 3.8-4.6(1H, br.), 3.98(2H, t, J=5.2Hz), 6.85-7.16(6H, m), 7.70(2H, d, J=7.7Hz), 8.59(1H, d, J=7.7Hz).
(13) 9-Chloro-2-phenyl-5,6-dihydro-4H-1,3-diazabenzo [e] azulen-1-ol
   Mass(APCI): m/z=311(M⁺+1),
   ¹H-NMR(CDCl₃) δ :1.62-1.82(2H, m), 2.2-2.4(2H, m), 2.48(2H, t, J=6.8Hz), 6.8-7.15(5H, m), 7.70(2H, d, J=6.9Hz), 8.24(1H, d, J=1.7Hz), 8.4-9.2(1H, br.).

### Example 3

A solution of isoamyl nitrite (0.85ml) in THF (1ml) was added dropwise to a solution of 3-methyl-6,7,8,9-tetrahydro-5H-benzo [a] cycrohepten-6-one (0.87g) in a mixed solvent of THF (10ml), water (2.5ml) and conc. hydrochloric acid (1.75ml) with stirring under cooling in an ice bath. The mixture was stirred under the same condition for 20 minutes and then at room temperature for one hour. The reaction mixture was neutralized with powdered sodium bicarbonate and extracted with ethyl acetate twice. The extracts were combined, washed with water and then dried over magnesium sulfate. The solvent was removed to give an oily intermediate, 3-methyl-6,7,8,9-tetrahydro-5H-benzo[a] cyclohepten-5-hydroxyimino-6-one (1.55g). This product was dissolved in 1,4-dioxane (20ml), and to the solution were added a solution of pyridine-2-aldehyde (589.1mg) in ethanol (6ml) and an aqueous solution (6ml) of ammonium acetate (1.16g). The mixture was refluxed with stirring for 2 hours, and the reaction mixture was distilled under vacuum. The resultant residue was dissolved in ethyl acetate and then washed with an aqueous solution of sodium bicarbonate and water. After drying over magnesium sulfate, the solvent was removed to give crude crystals. The crystals were washed with diisopropyl ether with stirring and then collected by filtration to give crystals of 2-(pyridin-2-yl)-5,6-dihydro-9-methyl-4H-1,3-diazabenzo [e] azulen-1-ol (0.57g).
Mass(APCI): m/z=292(M⁺+1);
¹H-NMR(DMSO-d₆) δ : 1.85-2.05(2H, m), 2.32(3H, s), 2.63-2.74(2H, m), 2.88(2H, t, J=7.3Hz), 7.02(1H, d, J=6.4Hz), 7.13(1H, d, J=7.7Hz), 7.4-7.5(1H, m), 7.98(1H, dt. J=1.7Hz, 7.9Hz), 7.8-8.3( 1H, br.), 8.65(1H, d, J=4.8Hz).

### Example 4

Following compounds (1) and (2) were prepared according to similar method to Example 3.
(1) 2-(Pyridin-3-yl)-5,6-dihydro-9-methyl-4H-1,3-diazabenzo [e] azulen-1-ol
   Mass(APCI): m/z=292(M⁺+1),
   ¹H-NMR(DMSO-d₆) δ : 1.9-2.05(2H, m), 2.23(3H, s), 2.6-2.75(2H, m), 2.85(2H, t, J=7.3Hz), 7.02(1H, d, J=7.7Hz), 7.15(1H, d, J=7.7Hz), 7.50(1H, dd, J=4.8Hz, 8.1Hz), 7.77(1H, br.s), 8.36(1H, br. s), 8.57(1H, dd, J=1.6Hz, 4.8Hz), 9.22(1H, s), 11.88(1H, s).
(2) 2-(Pyridin-4-yl)-5,6-dihydro-9-methyl-4H-1,3-diazabenzo [e] azulen-1-ol
   Mass(APCI): m/z=292(M⁺+1);
   ¹H-NMR(DMSO-d₆) δ : 1.9-2.05(2H, m), 2.34(3H, s), 2.62-2.72(2H, m), 2.85(2H, t, J=7.3Hz), 7.04(1H, d, J=7.7Hz), 7.17(1H, d, J=7.7Hz), 7.76(1H, br.s), 8.02(2H, br.s), 8.65(2H, d, J=6.2Hz), 12.01(1H, s).

### Example 5

3-Fluoro-5-hydroxyimino-6,7,8,9-tetrahydro-5H-benzo [a] cyclohepten-6-one (0.50g), pyridine-2-aldehyde (284.3mg) and ammonium acetate (558.0mg) were dissolved in a mixed solvent of 1,4-dioxane (10ml), ethanol (3ml) and water (3ml), and the solution was stirred at 100°C for 2 hours. The reaction mixture was cooled to room temperature and distilled under vacuum. To the resultant residue were added with stirring ethyl acetate and an aqueous solution of sodium bicarbonate. The organic layer was separated, washed with water twice and dried over magnesium sulfate. The solvent was removed under vacuum to give 9-fluoro-2-pyridin-2-yl)-5,6-dihydro-4H-1,3-diazabenzo [e] azulen-1-ol as crystals. The crystals were ground in diisopropyl ether and collected by filtration to give pure crystals (555.3mg, yield 78.0%).
Mass(APCI): m/z=296(M⁺+1);
¹H-NMR(DMSO-d₆) δ : 1.93-2.01 (2H, m), 2.71-2.77(2H, m), 2.94(2H, t, J = 7.0Hz), 7.04(1H, dt, J = 2.8Hz, 8.4Hz), 7.28(1H, t, J = 7.3Hz), 7.41-7.49(1H, m), 7.95-8.03(1H, m), 7.7-8.3(1H, br.), 8.68(1H, d, J=4.8Hz), 8.6-9.4(1H, br.), 13.13(1H, br.).

### Example 6

Following compounds (1) and (2) were prepared by similar methods to Example 5.
(1) 9-Fluoro-2-(pyridin-3-yl)-5,6-dihydro-4H-1,3-diazabenzo [e] azulen-1-ol
   Mass(APCI): m/z=296(M⁺+1);
   ¹H-NMR(DMSO-d₆) δ : 1.87-2.05 (2H, m), 2.65-2.77(2H, m), 2.90(2H, t, J = 7.3Hz), 7.04(1H, dt, J = 2.7Hz, 8.4Hz), 7.30(1H, dd, J =6.2Hz,8.4Hz), 7.52(1H, dd, J=4.8Hz, 8.0Hz), 7.72(1H, br.d, J=10.9Hz), 8.38(1H, br.d, J=8.3Hz), 8.59(1H, dd, J=1.5Hz, 4.8Hz), 9.21(1H, s), 12.02(1H, s).
(2) 9-Fluoro-2-(pyridin-4-yl)-5,6-dihydro-4H-1,3-diazabenzo[e]azulen-1-ol
   Mass(APCI): m/z=296(M⁺+1),
   ¹H-NMR(DMSO-d₆) δ : 1.9-2.05 (2H, m), 2.65-2.8(2H, m), 2.90(2H, t, J = 7.2Hz), 7.06(1H, dt, J = 2.7Hz, 8.4Hz), 7.31(1H, dd, J =6.2Hz,8.4Hz), 7.76(1H, br.), 8.04(2H, br.), 8.67(2H, dd, J=1.5Hz, 4.7Hz), 12.19(1H, br.s).

### Example 7

Phosphorus trichloride (0.57ml) was added to a solution of 9-methoxy-2-(pyridin-4-yl)-5,6-dihydro-4H-1,3-diazabenzo [e] azulen-1-ol (1.0g) in DMF (14ml) with stirring at room temperature. The mixture was stirred under the same condition for one hour. To the reaction mixture was added an aqueous solution of sodium bicarbonate, and the mixture was extracted with ethyl acetate twice. The extracts were combined, washed with water and dried over magnesium sulfate. The solvent was removed to give a mass (0.8g) of crystals. The mass was dissolved in method (20ml) and then hydrogenated in the presence of a catalytic amount of palladium on carbon and ammonium formate (1.0g) under reflux for 3 hours. The catalyst was filtered off, and methanol was removed under vacuum. To the residue was added an aqueous solution of sodium bicarbonate, and mixture was extracted with ethyl acetate twice. The extracts were combined, washed with water and dried over magnesium sulfate. The solvent was removed under vacuum, and the resultant mass of amorphous product was crystallized by treating with a mixture of chloroform and methanol (30:1). The crystals were collected by filtration to give 9-methoxy-2-(pyridin-4-yl)-3,4,5,6-tetrahydro-1,3-diazabenzo [e] azulene (595.8mg) as yellow crystalline powder.
Mass(APCI):m/z=292(M⁺+ 1);
¹H-NMR(DMSO-d₆) δ : 1.85-2.0(2H, m),2.75-2.95(2H, m),3.01(2H, t, J=6.4Hz), 3.80(3H,s), 6.69(1H, dd, J=2.7Hz, 8.2Hz), 7.05(1H, d, J=8.2Hz), 7.75(1H, d, 2.7Hz), 7.91(2H, d, J=6.0Hz), 8.00(1H, br.s), 8.65(1H, d, J=6.0Hz).

### Example 8

Following compounds (1)-(15) were prepared according to similar method to Example 7.
(1) 9-Methoxy-2-phenyl-3,4,5,6-tetrahydro-1,3-diazabenzo [e] azulene
   Mass(APCI): m/z=291(M⁺+1);
   ¹H-NMR(DMSO-d₆) δ : 1.8-2.0(2H, m), 2.75-2.85(2H, m), 2.9-3.05(2H, m), 3.79(3H, s), 6.7-8.2(9H, m), 12.76(1H, br).
(2) 9-Methoxy-2-(pyridin-3-yl)-3,4,5,6-tetrahydro-1,3-diazabenzo [e] azulene
   Mass(APCI): m/z=292(M⁺+1);
   ¹H-NMR(DMSO-d₆) δ : 1.8-2.0(2H, m), 2.73-2.86(2H, m), 2.9-3.05(2H, m), 3.78 and3.82(3H, each s(4:1)), 6.66 and 6.74(1H, each dd(4:1), J = 2.8Hz, 8.2Hz), 7.04 and 7.11(1H, each d(4: 1), J = 8.3Hz), 7.49(1H, dd, J=4.8Hz, 8.0Hz), 7.39 and 7.75(1H, each d(1:4), J=2.8Hz), 8.29 and 8.37(1H, each dt(4:1), J=8.0Hz,1.9Hz), 8.54(1H, dd, J=1.6Hz, 4.7Hz), 9.19 and 9.23(1H, each d(4:1), J=2.8Hz), 12.26 and 2.61(1H, each s(1:4)).
(3) 9-Methoxy-2-(pyridin-2-yl)-3,4,5,6-tetrahydro-1,3-diazabenzo [e] azulene
   Mass(APCI): m/z=292(M⁺+1);
   ¹H-NMR(DMSO-d₆) δ : 1.8-2.0(2H, m), 2.73-2.85(2H, m), 2.96(2H, t, J=6.5Hz), 3.78 and 3.84(3H, each s(5:1)), 6.65(1H, dd, J = 2.8Hz, 8.2Hz), 7.03(1H, d, J = 8.3Hz), 7.35-7.40(1H, m), 7.75(1H,d,J=2.8Hz), 7.85-7.83(1H, m), 8.12(1H, d, J=7.9Hz), 8.55-8.6(1H, m), 12.71(1H,s).
(4) 8-Methoxy-2-phenyl-4,5-dihydronaphtho [1,2-d] imidazole
   Mass(APCI): m/z=277(M⁺+1);
   ¹H-NMR(CDCl₃) δ : 2.8-3.1(4H, m), 3.77(3H, s), 6.65(1H, dd, J = 2.6Hz, 8.3Hz), 7.09-8.15(9H, m).
(5) 2-Phenyl-5,6-dihydro-4H-1,3-diazabenzo [e] azulene
   Mass(APCI): m/z=261(M⁺+1);
   ¹H-NMR(CDCl₃) δ : 1.95-2.1(2H, m), 2.83-3.05(4H, m), 7.1-7.45(7H, m), 7.7-8.0(1H, br.), 7.8-7.9(2H, m).
(6) 2-(Pyridin-2-yl)-5,6-dihydro-4H-1,3-diazabenzo [e] azulene
   Mass(APCI): m/z=262(M⁺+1);
   ¹H-NMR(DMSO-d₆) δ : 1.85-2.0(2H, m), 2.8-3.0(4H, m), 7.05-7.4(4H, m), 7.89(1H, dt, J=1.7Hz, 7.8Hz), 8.15(2H, dd, J=7.9Hz, 13.8Hz), 8.6(1H, d, J=4.7Hz), 12.72(1H, s).
(7) 2-(Pyridin-3-yl)-5,6-dihydro-4H-1,3-diazabenzo [e] azulene
   Mass(APCI-DV)/negative: m/z=260(M⁺-1);
   ¹H-NMR(DMSO-d₆) δ : 1.87-2.05(2H, m), 2.8-2.9(2H, m), 2.95-3.05(2H, m), 7.05-7.3(3H, m), 7.49(1H, dd, J=4.8Hz, 8.0Hz), 8.15(1H, br.s), 8.31(1H, d, J=8.0Hz), 8.54(1H, dd, J=1.5Hz, 4.8Hz), 9.18(1H, s), 12.61(1H, s).
(8) 2-(Pyridin-4-yl)-5,6-dihydro-4H-1,3-diazabenzo [e] azulene
   Mass(APCI-DV)/negative: m/z=260(M⁺-1);
   ¹H-NMR(DMSO-d₆) δ : 1.85-2.05(2H, m), 2.8-2.95(2H, m), 2.95-3.05(2H, m), 7.05-7.4(3H, m), 7.8-8.0(2H, m), 8.1-8.25(1H, br.s), 8.64(2H, d, J=4.6Hz), 12.79(1H, br.s).
(9) 2-(1,4-Benzodioxan-6-yl)-5,6-dihydro-4H-1,3-diazabenzo[e] azulene
   Mass(APCI): m/z=319(M⁺+1);
   ¹H-NMR(CDCl₃) δ : 1.95-2.1(2H, m), 2.86-3.05(4H, m), 4.27(4H, s), 6.85-7.36(7H, m), 7.78(1H, br.s).
(10) 2-( 1-Isopropyl-6-oxo-1,6-dihydropyridazin-3-yl)-5,6-dihydro-4H-1,3-diazab enzo [e] azulene
   Mass(APCI): m/z=321(M⁺+1);
   ¹H-NMR(CDCl₃) δ : 1.43(6H, d, J=6.6Hz), 2.0-2.2(2H, m), 2.85-3.15(4H, m), 5.38(1H, heptet, J=6.6Hz), 6.95-7.45(4H, m), 8.0-8.26(2H, m), 9.52(1H, br.s).
(11) 2-(Imidazol-3-yl)-5,6-dihydro-4H-1,3-diazabenzo [e] azulene
   Mass(APCI): m/z=251(M⁺+1);
   ¹H-NMR(CDCl₃) δ : 2.0-2.15(2H, m), 2.85-3.1(4H, m), 7.08-7.3(4H, m), 7.64(2H, s), 7.7-7.9(2H, br).
(12) 2-(Pyridin-2-yl)-5,6-dihydro-9-methyl-4H-1,3-diazabenzo [e] azulene
   Mass(APCI): m/z=276(M⁺+1);
   ¹H-NMR(DMSO-d₆) δ : 1.83-2.0(2H, m), 2.32(3H, s), 2.75-2.85(2H, m), 2.96(2H, t, J=6.5Hz), 6.85-7.08(2H, m), 7.35-7.4(1H, m), 7.8-7.95(1H, m), 8.0(1H, s), 8.1-8.15(1H, m), 8.57-8.62(1H, m), 12.69(1H, s).
(13) 2-(Pyridin-3-yl)-5,6-dihydro-9-methyl-4H-1,3-diazabenzo [e] azulene
   Mass(APCI): m/z=276(M⁺+1);
   ¹H-NMR(DMSO-d₆) δ : 1.85-2.03(2H, m), 2.33(3H, s), 2.75-2.85(2H, m), 2.99(2H, t, J=6.5Hz), 6.90(1H, d, J=7.7Hz), 7.02(1H, d, J=7.7Hz), 7.45-7.52(1H, m), 7.96(1H, br.s), 8.31(1H, d, J=7.8Hz), 8.54(1H, dd, J=1.6Hz, 4.8Hz), 9.18(1H, d, J=1.6Hz), 12.57(1H, br.s).
(14) 2-(Pyridin-4-yl)-5,6-dihydro-9-methyl-4H-1,3-diazabenzo [e] azulene
   Mass(APCI): m/z=276(M⁺+1);
   ¹H-NMR(DMSO-d₆) δ : 1.85-2.03(2H, m), 2.33(3H, s), 2.75-2.88(2H, m), 3.00(2H, t, J=6.5Hz), 6.92(1H, d, J=7.5Hz), 7.03(1H, d, J=7.5Hz), 7.92(3H, d, J=5.8Hz), 8.63(2H, dd, J=1.6Hz, 4.6Hz), 12.75(1H, br.s).
(15) 2-Phenyl-4,5-dihydro-3H[1] benzoxepino [4,5-d] imidazole
   Mass(APCI): m/z=263(M⁺+1);
   ¹H-NMR(CDCl₃) δ : 3.25(2H, t, J=5.2Hz), 4.36(2H, t, J=5.2Hz), 7.03-8.01(10H, m).

### Example 9

Phosphorus trichloride (0.32ml) was added to a solution of 9-fluoro-2-(pyridin-2-yl)-5,6-dihydro-4H-1,3-diazabenzo [e] azulen-1-ol (540.5mg) in DMF (7.5ml) with stirring under cooling in an ice bath. The mixture was stirred under the same condition for 0.25 hour and then at room temperature for 3.5 hours. Water was poured into the reaction mixture. The mixture was made basic with sodium bicarbonate with stirring and extracted with ethyl acetate twice. The extracts were combined, washed with water and saline and then dried over magnesium sulfate. The solvent was removed under vacuum to give brown colored oily product (0.38g). This product was dissolved in methanol (20ml). To the methanol solution were added ammonium formate (0.58g) and 10% palladium on carbon (0.1g), and the mixture was refluxed with stirring for 3 hours. The catalyst was filtered off with Celite, and the filtrate was distilled under vacuum. To the residue were added with stirring an aqueous solution of sodium bicarbonate and ethyl acetate. The organic layer was separated, washed with water and saline and then dried over magnesium sulfate. The solvent was removed under vacuum to give yellow oily product (0.37g). This product was subjected to column chromatography on silica gel eluting with a mixed solvent of chloroform and methanol (50:1). Fractions containing the desired product were combined and distilled under vacuum to give an amorphous mass (0.37g). The mass was ground in diisopropyl ether, and the resultant crystals were collected by filtration and dried to give 9-fluoro-2-(pyridin-2-yl)-5,6-dihydro-4H-1,3-diazabenzo [e] azulene (167.3mg).
Mass(APCI): m/z=280(M⁺+1);
¹H-NMR(DMSO-d₆) δ : 1.85-2.0(2H, m), 2.75-3.05(4H, m), 6.81-6.94(1H, m), 7.1-7.2(1H, m), 7.32-7.42(1H, m), 7.84-7.94(2H, m), 8.1-8.17( 1H, m), 8.57-8.64(1H, m), 12.82(1H, s).

### Example 10

Following compounds (1) and (2) were prepared by similar method to Example 9.
(1) 9-Fluoro-2-(pyridin-3-yl)-5,6-dihydro-4H-1,3-diazabenzo [e] azulene.
   Mass(APCI): m/z=280(M⁺+1);
   ¹H-NMR(DMSO-d₆) δ : 1.9-2.05 (2H, m), 2.8-2.95(2H, m), 2.98(2H, t, J = 6.3Hz), 6.85-7.0(1H,m), 7.1-7.25(1H, m),7.50(1H, dd, J=4.8Hz, 8.0Hz), 7.85(1H, d, J=11.7Hz), 8.30(1H, d, J=8.2Hz), 8.55(1H, d, J=4.8Hz), 9.17(1H, s), 12.70(1H, br.s).
(2) 9-Fluoro-2-(pyridin-4-yl)-5,6-dihydro-4H-1,3-diazabenzo [e] azulene.
   Mass(APCI): m/z=280(M⁺+1);
   ¹H-NMR(DMSO-d₆) δ : 1.9-2.0 (2H, m), 2.8-3.1(4H, m), 6.85-7.0(1H,m), 7.1-7.25(1H, m), 7.85-7.95(3H, m), 8.64(2H, dd, J=1.48Hz, 4.6Hz), 12.88(1H, br.s).

### Example 11

Phosphorus trichloride (3.37ml) was added dropwise to a solution of 9-chloro-2-phenyl-5,6-dihydro-4H-1,3-diazabenzo [e] azulen-1-ol (6.0g) in DMF (80ml) with stirring under cooling in an ice bath. The mixture was stirred under the same condition for 0.5hour and then at room temperature for 1.5hours. the mixture was poured into water, neutralized with powdered sodium bicarbonate and extracted with ethyl acetate twice. The extract was washed with water and dried over magnesium sulfate, and the solvent was removed under vacuum to give an oily product. The product was crystallized by treating with ethyl acetate, and the resultant crystals were collected by filtration and purified by subjecting to column chromatography on silica gel eluting with a mixture of chloroform and methanol (100:1). Fractions containing the desired product were combined and distilled under vacuum. The resultant crystals (3.86g) were washed with chloroform to give 9-chloro-2-phenyl-5, 6-dihydro-4H-1,3-diazabenzo [e] azulene (2.34g).
Mass(APCI): m/z=295(M⁺+1);
¹H-NMR(CDCl₃) δ : 1.95-2.03(2H, m), 2.8-2.93(2H, m), 3.03(2H, t, J=6.7Hz),7.0-7.15(2H, m), 7.35-7.55(4H, m), 7.8-7.95(2H,m), 7.8-8.3(1H, br.).

### Example 12

1M-Solution of phosphorus trichloride in dichloromethane (16ml = 4 molar equivalent) was added dropwise to a suspension of 9-methoxy-2-(pyridin-4-yl)-5,6-dihydro-4H-1,3-diazabenzo [e] azulene (1.16g) in dichloromethane (5ml) with stirring at room temperature. The mixture was refluxed with stirring for 3 hours. To the reaction mixture were added with stirring an aqueous solution saturated with sodium bicarbonate and ethyl acetate. The resultant orange colored precipitates were filtered off and the separated organic layer was washed with water twice and dried over magnesium sulfate. The solvent was removed under vacuum, and the resultant crystalline powder was subjected to column chromatography on silica gel eluting with a mixture of chloroform and methanol (20:1). Fractions containing the desired product were combined and distilled under vacuum. The resultant mass of crystals were washed with methanol with stirring to give crystals of 9-hydroxy-2-(pyridin-4-yl)-5,6-dihydro-4H-1,3-diazabenzo [e] azulene (428.4mg).
Mass(APCI): m/z=278(M⁺+1);
¹H-NMR(DMSO-d₆) δ : 1.85-2.0(2H, m), 2.7-2.85(2H, m), 2.9-3.05(2H, m), 6.49(1H, dd, J=2.7Hz, 8.1Hz), 6.91(1H, d, J=8.1Hz), 7.64(1H, d, J=2.7Hz), 7.87(2H, dd, J=1.6Hz, 4.6Hz), 8.6-8.65(2H, m), 9.08(1H, s), 12.74(1H, s).

### Test Example 1

Effects on binding ability of PYY(peptide YY) to HEK(human embryo kidneys)-293 cells transducted with human NPY(neuropeptide Y) Y5 receptors

HEK-293 cells, which stably manifest human NPY Y5 receptors, were incubated at 25 °C for 120 minutes in the absence of test compounds in an assay medium containing 0.1 mM PMSF (phenylmethylsulfonyl fluoride), 0.05% bacitracin, 0.1% BSA (bovine serum albumin) and 25mM Tris HCl (pH 7.4). The non-specific binding was measured in the presence of 10⁻⁶M non-labelled PYY. The reaction was terminated by rapidly filtering with Wattmann GF/C glass fiber filter with using the cell harvester. After the glass fiber filter was washed with 150 mM sodium chloride and 25 mM Tris HCl buffer solution (pH 7.4) containing 0.01% BSA, radioactivity of the test compound was measured by using the γ -counter. The IC₅₀ value of the test compound was determined by the inhibition of [¹²⁵I]-PYY binding obtained by the test compound at various concentrations. The results are shown in Table 1. The test compound is represented with the Example number in which the compound was obtained.

**Table 1**

| Test compounds | IC₅₀ (M) | Test compounds | IC₅₀ (M) |
|---|---|---|---|
| 2 (1) | 2.4×10⁻⁸ | 8 (6) | 1.3×10⁻⁷ |
| 1 | 7.3×10⁻⁸ | 8 (7) | 7.3×10⁻⁹ |
| 8 (1) | 2.5×10⁻⁸ | 8 (8) | 2.0×10⁻⁸ |
| 7 | 1.3×10⁻⁸ | 8 (12) | 3.2×10⁻⁸ |
| 8 (2) | 6.3×10⁻⁹ | 8 (13) | 2.3×10⁻⁹ |
| 8 (3) | 1.2×10⁻⁷ | 8 (14) | 6.4×10⁻⁹ |
| 8 (4) | 5.1×10⁻⁸ | 9 | 4.6×10⁻⁸ |
| 8 (5) | 5.8×10⁻⁹ | 10 (1) | 6.9×10⁻⁹ |
| 8 (10) | 2.6×10⁻⁷ | 10 (2) | 1.3×10⁻⁸ |
| 8 (11) | 5.5×10⁻⁷ | 8 (15) | 6.1×10⁻⁸ |
| 8 (9) | 1.8 × 10⁻⁷ | 11 | 1.7×10⁻⁹ |

### Possibility of industrial utility

The fused imidazole derivatives (I) and their salts have antagonism against NPY receptors, especially against NPY-Y5 receptors, and are useful for treatment and prevention of diabetes, hyperlipemia, hypertension and arteriosclerosis.

## Claims

1. A fused imidazole derivatives represented by the general formula: 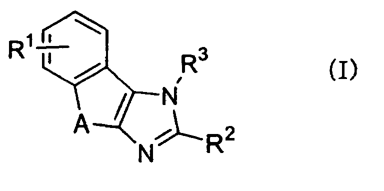 wherein
R¹ is a hydrogen atom, a halogen atom, hydroxy group, a lower alkyl group or a lower alkoxy group,
R² is an aryl group, benzodioxanyl group, or 5-6 membered, monocyclic, unsaturated, heterocyclic group containing nitrogen atom(s) which may be substituted with lower alkyl, trityl or oxo,
R³ is a hydrogen atom or hydroxy group,
A is a group represented by the formula : -(CH₂)ₘ - or -O-(CH₂)ₘ- [wherein m is an integer of 1-3]
and its salt.

2. The fused imidazole derivative and its salt according to Claim 1, wherein the monocyclic, unsaturated, heterocyclic group containing nitrogen atom(s) is a monocyclic, unsaturated, heterocyclic group containing 1 or 2 nitrogen atoms.

3. The fused imidazole derivative and its salt according to Claim 2, wherein the monocyclic, unsaturated, heterocyclic group is imidazolyl group, pyridyl group or pyridazinyl group.

4. The fused imidazole derivatives and its salt according to any one of Claims 1-3, wherein A is a group represented by the formula: the -(CH₂)₂-, - (CH₂)₃- or -O-(CH₂)₂-.

5. The fused imidazole derivative and its salt according to Claim 4, wherein A is a group represented by the formula: -O-(CH₂)₂-. R¹ is a hydrogen atom, and R² is an aryl group.

6. A pharmaceutical composition containing a fused imidazole derivative or its salt according any one of Claims 1-5 as an effective ingredient.

7. The pharmaceutical composition according to Claim 6, wherein the composition is a medicament for preventing or treating obesty, ingestion disorder, diabetes, hyperlipemia, hypertension and/or arteriosclerosis.
